(19) **Europäisches Patentamt**

**European Patent Office**

**Office européen des brevets**

(11) **EP 0 695 286 B1**

(12) **EUROPÄISCHE PATENTSCHRIFT**

(45) Veröffentlichungstag und Bekanntmachung des Hinweises auf die Patenterteilung:
07.01.1998  **Patentblatt 1998/02**

(51) Int Cl.⁶: **C07C 53/06**, C07C 53/10, C07C 51/41

(21) Anmeldenummer: **94914402.6**

(22) Anmeldetag: **19.04.1994**

(86) Internationale Anmeldenummer:
**PCT/EP94/01202**

(87) Internationale Veröffentlichungsnummer:
**WO 94/25422 (10.11.1994 Gazette 1994/25)**

(54) **VERFAHREN ZUR HERSTELLUNG VON CÄSIUMSALZEN AUS CÄSIUM-ALUMINIUM-ALAUN**

METHOD OF PREPARING CAESIUM SALTS FROM CAESIUM ALUMINIUM ALUM

PROCEDE DE FABRICATION DE SELS DE CESIUM A PARTIR D'ALUN DE CESIUM-ALUMINIUM

(84) Benannte Vertragsstaaten:
**AT CH DE DK FR GB LI LU**

(30) Priorität: **24.04.1993  DE 4313480**

(43) Veröffentlichungstag der Anmeldung:
**07.02.1996  Patentblatt 1996/06**

(73) Patentinhaber: **METALLGESELLSCHAFT AG**
**60323 Frankfurt am Main (DE)**

(72) Erfinder:
• **HOFMANN, Hartmut**
 **D-65812 Bad Soden (DE)**

• **KÖBELE, Klaus**
 **D-63128 Dietzenbach (DE)**
• **PRINZ, Horst**
 **D-61169 Friedberg (DE)**
• **PHILLIPP, Bernd**
 **D-38685 Langelsheim (DE)**
• **HARMS, Gerd**
 **D-38640 Goslar (DE)**
• **SCHIEDT, Alexander**
 **D-55131 Mainz (DE)**
• **HECKTOR, Ulrike**
 **D-61169 Friedberg (DE)**

(56) Entgegenhaltungen:
**DE-B- 1 253 252**       **US-A- 3 207 571**

**Beschreibung**

Die Erfindung betrifft ein Verfahren zur Herstellung von Cäsiumsalzen aus Cäsium-Aluminium-Alaun.

Aus der US-PS 3 489 509 ist ein Verfahren zur Gewinnung von Cäsiumverbindungen in hoher Reinheit bekannt. Cäsium-Aluminium-Alaun wird mit Bariumhydroxid behandelt, anschließend einer Reaktion mit Kohlendioxid unterzogen, und schließlich wird durch Zugabe der stöchiometrischen Menge an Bariumhalogenid das reine Cäsiumhalogenid gewonnen. Ungünstig für die Gewinnung des Cäsiumhalogenids aus dem Cäsium-Aluminium-Alaun ist dabei, daß der Einsatz von Bariumhydroxid und Bariumhalogeniden erhebliche Kosten verursacht sowie daß eine Neutralisation durch Umsetzung mit Kohlendioxid erfolgen muß.

Aufgabe der Erfindung ist es, ein Verfahren zu schaffen, mit dem die beschriebenen Nachteile vermieden werden können.

Erfindungsgemäß wird die Aufgabe gelöst, indem Cäsium-Aluminium-Alaun mit der dem Aluminium äquimolaren Menge an Calciumhydroxid und mit der dem Cäsium äquimolaren Menge eines gut wasserlöslichen Calciumsalzes in einer Eintopfreaktion in Gegenwart von Wasser umgesetzt wird und das ausfallende Aluminiumhydroxid sowie das ausfallende Calciumsulfat durch Filtration oder Zentrifugation abgetrennt werden. Neben dem Verzicht auf die im Vergleich zu den Calciumverbindungen teuren Bariumverbindungen und dem Wegfall der Neutralisationsstufe mit Kohlendioxid hat das erfindungsgemäße Verfahren den Vorteil, daß durch die direkte Umsetzung Cäsiumsalzlösungen im Konzentrationsbereich > 10 % erhalten werden. Dadurch wird die Wasser-Abtrennarbeit bei der Herstellung konzentrierter Cäsiumsalzlösungen bzw. von festen Salzen erheblich reduziert.

Die an sich naheliegende direkte Umsetzung von Cäsium-Aluminium-Alaun mit der stöchiometrischen Menge an Calciumhydroxid gemäß der Gleichung

$$CsAl(SO_4)_2 + 2Ca(OH)_2 \rightarrow CsOH + Al(OH)_3 + 2\ CaSO_4$$

führt nicht zu zufriedenstellenden Ausbeuten an Cäsiumhydroxid, da der sich bildende Niederschlag aus Aluminiumhydroxid und Gips wahrscheinlich suspendiertes Calciumhydroxid oberflächlich maskiert und damit der Reaktion entzieht. Ein Überschuß an Calciumhydroxid, der die maskierte Calciumhydroxidmenge ausgleicht, kann jedoch nicht so genau dosiert zugesetzt werden, so daß eine schlecht zu entfernende Verunreinigung des gewünschten Produkts Cäsiumhydroxid mit Calciumhydroxid eintritt. Deshalb wird nach dem Stand der Technik das zwar besser lösliche, jedoch wesentlich teurere Bariumhydroxid verwendet.

Überraschenderweise wird es durch das erfindungsgemäße Verfahren möglich, die reinen Cäsiumsalze in einer Eintopfreaktion herzustellen. In vorteilhafter Weise werden als gut wasserlösliche Calciumsalze Calciumformiat, -acetat, -citrat, -chlorid oder -bromid eingesetzt.

Die Herstellung der Cäsiumsalze aus Cäsium-Aluminium-Alaun erfolgt in bevorzugter Weise so, daß die Umsetzung im Temperaturbereich von 80 bis 120°C bei einer Suspensionsdichte durchgeführt wird, die bei Reaktionsbeginn 100 bis 500 g unlöslicher Feststoff pro l Wasser beträgt. Der unslösliche Feststoff besteht aus den bei Reaktionsbeginn unlöslichen Teilen des Ca$(OH)_2$, des Calciumsalzes und des Cäsium-Aluminium-Alauns.

Durch nachfolgende Beispiele wird die Erfindung näher erläutert.

Beispiel 1: Herstellung von Cäsiumformiat

Ein beheizbarer Rührbehälter mit einem Volumen von 500 l wurde mit 290 l Wasser, 17 kg Calciumoxid und 13 kg Calciumformiat beschickt. Die Suspension wurde unter Rühren zum Sieden erhitzt und 111 kg Cäsium-Aluminium-Alaun zugesetzt. Das Reaktionsgemisch wurde 2 Stunden bei Siedehitze gerührt. Dann ließ man das Reaktionsgemisch abkühlen und filtrierte es. Das Filtrat enthielt ca. 11 % Cäsiumformiat, 0,1 % Calcium-, 0,05 % Aluminium- und 1 % Sulfationen. Der Filtrationsrückstand wurde mit Wasser nachgewaschen. Es wurden etwa 95 % des eingesetzten Cäsiums in Cäsiumformiat umgewandelt und in den vereinigten Filtraten nachgewiesen.

Beispiel 2: Herstellung von Cäsiumacetat

Eine Apparatur gemäß Beispiel 1, aber mit einem Volumen von 1000 l, wurde mit 375 l Wasser, 43,8 kg Calciumoxid und 45,8 kg Calciumacetat beschickt. Die Suspension wurde bis zum Sieden erhitzt und 296 kg Cäsium-Aluminium-Alaun zugesetzt. Anschließend wurde 2 Stunden bei Siedehitze gerührt, Abkühlen lassen und filtriert. Es resultierte eine ca. 20 %ige Cäsiumacetatlösung, die 58 % des eingesetzten Cäsiums enthielt. Durch Nachwaschen des Filtrationsrückstandes wurden weitere 37 % des eingesetzten Cäsiums als wäßrige Cäsiumacetatlösung gewonnen.

**Patentansprüche**

1. Verfahren zur Herstellung von Cäsiumsalzen aus Cäsium-Aluminium-Alaun, dadurch gekennzeichnet, daß Cäsium-Aluminium-Alaun mit der dem Aluminium äquimolaren Menge an Calciumhydroxid und mit der dem Cäsium äquimolaren Menge eines gut wasserlöslichen Calciumsalzes in einer Eintopfreaktion in Gegenwart von Wasser umgesetzt wird und das ausfallende Aluminiumhydroxid sowie das ausfallende Calciumsulfat durch Filtration oder Zentrifugation abgetrennt werden.

2. Verfahren nach Anspruch 1, dadurch gekennzeichnet, daß als gut wasserlösliches Calciumsalz Calciumformiat, -acetat, -citrat, -chlorid oder -bromid eingesetzt wird.

3. Verfahren nach den Ansprüchen 1 und 2, dadurch gekennzeichnet, daß die Umsetzung im Temperaturbereich von 80 bis 120°C bei einer Suspensionsdichte durchgeführt wird, die bei Reaktionsbeginn 100 bis 500 g unlöslicher Feststoff pro l Wasser beträgt.

## Claims

1. A method for the production of caesium salts from caesium-aluminium-alum, characterised in that caesium-aluminium-alum is reacted with the quantity of calcium hydroxide which is equimolar to the aluminium and with the quantity of a readily water-soluble calcium salt which is equimolar to the caesium in a single-vessel reaction in the presence of water and the aluminium hydroxide precipitated and the calcium sulphate precipitated are separated off by filtration or centrifuging.

2. A method according to Claim 1, characterised in that calcium formate, calcium acetate, calcium citrate, calcium chloride or calcium bromide is used as the readily water-soluble calcium salt.

3. A method according to Claims 1 and 2, characterised in that the reaction is performed in a temperature range from 80 to 120°C at a suspension density which is 100 to 500 g insoluble solids per litre of water at the start of the reaction.

## Revendications

1. Procédé de préparation de sels de césium à partir d'alun de césium-aluminium, caractérisé en ce qu'il consiste à mettre à régir l'alun de césium-aluminium sur la quantité d'hydroxyde de calcium qui est équimolaire à l'aluminium et sur la quantité d'un sel de calcium bien soluble dans l'eau qui est équimolaire au césium dans une réaction dans un seul vase en présence d'eau et à séparer par filtration ou par centrifugation l'hydroxyde d'aluminium qui précipite ainsi que le sulfate de calcium qui précipite.

2. Procédé suivant la revendication 1, caractérisé en ce qu'il consiste à utiliser comme sel de calcium bien soluble dans l'eau du formiate de calcium, de l'acétate de calcium, du citrate de calcium, du chlorure de calcium ou du bromure de calcium.

3. Procédé suivant la revendication 1 ou 2, caractérisé en ce qu'il consiste à effectuer la réaction dans une plage de température de 80 à 120°C avec une masse volumique de la suspension qui au début de la réaction est de 100 à 500 g de matière solide insoluble par litre d'eau.